# EUROPÄISCHE PATENTSCHRIFT

(11) **EP 1 319 374 B1**
(45) Veröffentlichungstag und Bekanntmachung des Hinweises auf die Patenterteilung: **01.02.2006**
(21) Anmeldenummer: 02027746.3
(22) Anmeldetag: 11.12.2002
(51) Int. Cl.: A61C 8/00, A61B 17/16

(54) **Formschraube für Knochenimplantate**
Thread-forming screw for bone implants
Vis taraudeuse pour implants osseux

(30) Priorität: 13.12.2001 DE 20120205 U
(43) Veröffentlichungstag der Anmeldung: 18.06.2003
(73) Patentinhaber: Dinkelacker, Wolfgang, Dr. med. dent., 71063 Sindelfingen (DE)
(72) Erfinder: Dinkelacker, Wolfgang, Dr. med. dent., 71063 Sindelfingen (DE)
(74) Vertreter: Kindermann, Manfred

(56) Entgegenhaltungen:
- EP-A- 0 000 549
- EP-A- 0 997 112
- DE-U- 20 113 183
- US-A- 5 427 527
- US-A- 5 642 996
- US-A- 5 772 437
- US-A- 5 961 328

## Beschreibung

### Bereich der Erfindung

Die Erfindung bezieht sich auf eine Formschraube zur Vorbereitung von Implantatlagern in menschlichen Knochen.

### Stand der Technik

Bei Knochenimplantaten ist die Einpassung des Implantats in den Knochen von entscheidender Bedeutung für den Einheilerfolg. Um ein an die Form eines rotationssymmetrischen Implantats angepaßtes Implantatlager im Knochen zu erzeugen, wird zweistufig vorgegangen. In einem ersten Schritt wird ein Loch vorgebohrt, dessen Durchmesser kleiner ist als der kleinste Implantatdurchmesser. In einem zweiten Schritt wird die Bohrung des ersten Schrittes durch Nachbohren in die dem Implantat entsprechende Form gebracht. Die USA Patentschrift 4,185,383 offenbart ein Dentalimplantat, das einen über die Längsachse abgestuften Zylinder aufweist. Zum Erzeugen einer an das Implantat angepaßten Bohrung wird mit einem Vorbohrwerkzeug ein kegelförmiges Loch in den Kieferknochen gebohrt. Dieses Loch wird durch ein entsprechend geformtes Nachbohrwerkzeug an die Form des Implantats angepaßt. Hierzu weist das Nachbohrwerkzeug ebensolche zylindrische Abstufungen auf wie das Implantats auf, so dass eine Bohrung hoher Genauigkeit erzeugt werden kann. Beide Bohrvorgänge werden vorzugsweise von Hand ausgeführt unter entsprechender Wahl von Bohrdruck und Drehzahl, so dass Gewebeverbrennungen vermieden werden.

Auch die EP 0 554 915 A1 offenbart ein abgestuftes Räumwerkzeug nach dem oberbegriff des Anspruchs 1, mit dem profilierte Öffnungen im Knochen hergestellt werden. In einem weiteren Schritt werden mit Hilfe eines Gewindeschneidwerkzeugs, das an das Profil der vorgeformten Öffnung und an ein in die Öffnung einzusetzendes schraubenförmiges Implantat angepasst ist, Gewindeabschnitte in die vorgeformte Öffnung geschnitten.

DE 296 06 335 U1 offenbart einen kieferchirurgischen Instrumentensatz zur Schaffung von Kieferöffnungen zum Einsetzen von Implantaten. Der Instrumentensatz umfasst wenigstens einen Vorformer und mehrere Implantatbettformer. Der Vorformer weist einen langgezogenen Schaft mit einem Griff und einer konischen Spitze auf, die an ihrem Ende eine konkave Kalotte besitzt. Die Implantatbettformer sind ähnlich ausgebildet und weisen an einer Arbeitsspitze Abstufungen auf, in denen sich der Durchmesser zum Ende hin stufenförmig verkleinert. Die Implantatbettformer weisen unterschiedliche Größen auf entsprechend der Größe der Implantate. Beim Gebrauch des Vorformers wird zunächst durch axialen Druck eine konische Öffnung im Oberkiefer geschaffen, wobei durch die Kalotte am Grunde der Öffnung ein linsenförmiges Knochenmaterial stehen bleibt, das anschließend beim Eindringen des Implantatbettformers durch dessen Spitze verdrängt und komprimiert wird. Durch die Abstufungen an der Spitze des Implantatbettformers werden die Knochenmaterialbereiche der zuvor vom Vorformer erzeugten konischen Öffnung verdrängt und in ein entsprechendes Stufenprofil zur Aufnahme eines abgestuften Implantats umgeformt. Ringförmige Markierungen am Schaft von Vorformer und Implantatbettformer dienen als Tiefenanzeige für die herzustellenden Öffnungen. Diese Instrumente sind zur Herstellung und Formung von Öffnungen im Oberkiefer bestimmt.

DE 295 22 073 U1 offenbart ein Werkzeug zum Präparieren von Hohlräumen in der Knochenspongiosa für die Aufnahme von Knochenimplantaten. Das Werkzeug umfasst einen leicht konischen Gewindeteil und einen Kopf zum Drehen des Werkzeugs. Hierbei sind die Konizität, die Form und Neigung der Gewindeflanken, die Übergangsradien zwischen benachbarten Gewindeflanken und die Oberflächenbeschaffenheit des Gewindeteil auf das in den Hohlraum einzusetzende Implantat sowie darauf abgestellt, dass bei der Einprägung der Gewindeform in das Wandmaterial des Knochenhohlraumes dieses radial nach außen verdrängt wird und möglichst wenig Knochenmaterial abgetragen wird.

### Zusammenfassung der Erfindung

Durch die Erfindung wird eine Formschraube für Knochenimplantate angegeben, durch die eine verbesserte Vorbereitung von passgenauen Öffnungen im menschlichen Knochen für die Aufnahme von Implantaten erreicht wird. Die Formschraube wird vor dem Einsetzen des Implantats in eine vorgebohrte Öffnung in den Knochen eingeschraubt zur Herstellung eines passgenauen Lagers für das Implantat: Die Formschraube weist einen Schaft auf, der in Abschnitte unterteilt und kopfseitig zum Eingriff mit einem Drehwerkzeug eingerichtet ist.

Die erfindungsgemäße Formschraube, wie sie in den Ansprüchen gekennzeichnet wird, ist in wenigstens einem von fußseitigen Abschnitten (7) mit selbstschneidendem Gewinde (10, 14, 15) versehen und wenigstens ein weiterer Abschnitt ist als Formkegel ausgebildet, dessen Grundfläche zum Schraubenkopf gerichtet ist und der beim Einschrauben das den Schaft der Formschraube umgebende Knochengewebe verdichtet und erweitert.

Erfindungsgemäß umfasst ihr Schaft einen ersten zylindrischen Abschnitt, der dem Fuß der Formschraube benachbart ist und der ein selbstschneidendes Gewinde aufweist. An den ersten Abschnitt schließt sich wenigstens ein weiterer zylindrishers Abschnitt an, dessen Durchmesser größer ist als der des ersten Abschnitts und der ebenfalls ein Gewinde aufweist, das wenigstens über einen Teil des weiteren Abschnitts selbstschneidend ausgebildet ist. In Richtung des Schraubenkopfes folgt wenigstens ein Formkegel, der den Schaft zum Schraubenkopf hin vergrößert. Am Schraubenkopf befindet sich ein Zapfen, der zum Eingriff mit einem Drehwerkzeug ausgebildet ist.

Durch die erfindungsgemäße Formschraube wird im menschlichen Knochen ein Implantatlager erzeugt, das eine hohe Passgenauigkeit aufweist und das das Implantatlager umgebende Knochengewebe verdichtet. Das durch die erfindungsgemäße Formschraube vorbehandelte Knochengewebe ermöglicht eine enge Verbindung zwischen Knochen und dem Implantat, das nach Entfernen der Formschraube in das so vorbehandelte Loch im Knochen eingebracht wird. Hierdurch werden von Anfang an ein sicherer Sitz des Implantats erreicht und ein problemloses Einheilen begünstigt.

### Beschreibung der Zeichnungen

Nachfolgend sind verschiedene Ausführungsformen der Erfindung anhand von Zeichnungen dargestellt. Es zeigen:
Figur 1 eine Seitenansicht auf eine Ausführungsform einer Formschraube gemäß der Erfindung;
Figur 2 eine Draufsicht auf die Formschraube von Figur 1 von der Kopfseite; und
Figur 3 eine Ansicht der Formschraube von Figur 1 von der Fußseite.

### Detaillierte Beschreibung des in den Zeichnungen dargestellten Ausführungsbeispiels der Erfindung

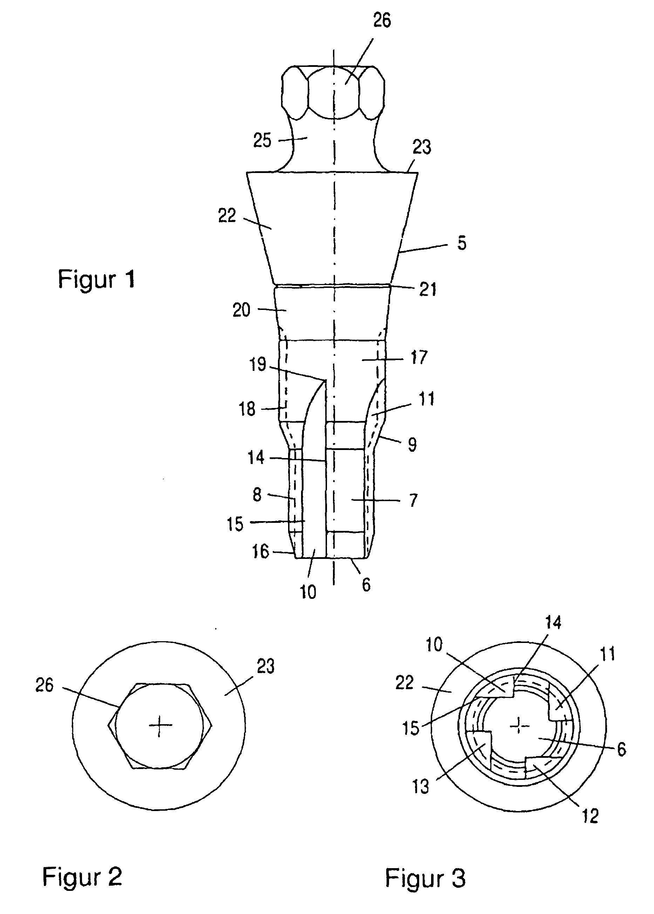

Die in Figur 1 dargestellte Formschraube, die vorzugsweise aus chirurgischem Stahl oder einer Titanlegierung besteht, weist einen rotationssymmetrischen, in Abschnitte unterteilten Schaft 5 auf. Dem Fuß 6 der Formschraube benachbart ist ein erster zylindrischer Abschnitt 7, dessen Umfang besitzt ein Schraubengewinde 8, das sebstschneidend ausgebildet ist. Hierzu sind am Umfang dieses Abschnitts wenigstens zwei, vorzugsweise aber vier längsseits verlaufende Nuten 10, 11, 12 und 13 angeordnet, die ein asymmetrisches Profil aufweisen. Diese Nuten 10-13 verlaufen im dargestellten Beispiel axial, sie können aber auch wendelförmig verlaufen. Jede dieser Nuten weist eine Schnittkante 14 und eine Auslaufkante 15 auf. Am Fuß 6 ist eine angeschrägte Phase 16 angebracht, die das Einsetzen der Formschraube in ein vorgebohrtes Loch im Knochen erleichtert.

An den ersten Abschnitt 7 schließt sich ein zweiter zylindrischer Abschnitt 17 an, der einen größeren Durchmesser aufweist als der Abschnitt 7 und der ebenfalls mit einem Schraubengewinde 18 ausgestattet ist. Das Gewinde 8 des ersten Abschnitts geht über einen abgeschrägten Übergangsbereich 9 in das Gewinde 18 des zweiten Abschnitts 17 über. Hierbei befinden sich die Gewindegänge auch auf den Übergangsbereich 9. Die Schnittnuten 10-13 erstrecken sich über den Übergangsbereich 9 und wenigstens einen Teil des zweiten Abschnitts 17 und laufen bei 19 in dessen Umfang aus.

Auf den zweiten zylindrischen Abschnitt folgen in Richtung des Schraubenkopfes zwei kegelförmige Abschnitte 20 und 22, welche die Funktion von Formkegeln haben, indem sie beim Einschrauben in den Knochen diesen verdichten und erweitern. Die zwei aufeinanderfolgenden Formkegel (20, 22) weisen eine unterschiedliche Steigung auf. An den Abschnitt 17 schließt sich ein erster Formkegel 20 an, in dem das Gewinde 17 ausläuft. Der Formkegel 20 ist durch eine schmale Umfangsnut 21 von einem zweiten Formkegel 22 getrennt, der einen größeren Neigungswinkel als der Formkegel 20 aufweist. Die Größe des Neigungswinkels der Formkegel 20, 22 hängt von der Art des Implantats ab.

Die Grundfläche des Formkegels 22 wird durch einen kopfseitigen Bund 23 gebildet, so dass sich der Durchmesser des Schafts 5 über die Länge der Kegels 20 und danach über die Länge des Kegels 22 jeweils mit unterschiedlicher Steigung vergrößert. Der Bund 23 geht in einen axialen Zapfen 25 über, an dessen Ende ein Sechskant 26 angeordnet ist, der den Kopf der Formschraube bildet.

Die Formschraube wird in eine vorgebohrtes zylindrisches Loch im Knochen eingesetzt und mittels eines Drehwerkzeugs, das am Sechskant 26 angreift, in den Knochen eingeschraubt. Hierbei schneiden die Schnittkanten 14 der Nuten 10-13 Gewindegänge in das Knochengewebe, das die Abschnitte 7 und 17 des Schaftes 5 umgibt. Das selbstschneidende Gewinde stellt einen konstanten Bohrvorschub sicher. Für die Drehung der Schraube kann ein Motorantrieb mit regelbarer Drehzahl verwendet werden.

Mit zunehmender Einschraubtiefe wird das Loch durch die Wirkung der Schnittkanten 14 im Übergangsbereich 9 und im Gewindeabschnitt 17 vergrößert. Beim weiteren Einschrauben wird das den Schaft 5 umgebende Knochengewebe zunächst durch den Formkegel 20 erweitert und durch Preßformung verdichtet. Mit einer Fortsetzung des Einschraubvorganges wird das den Schaft 5 umgebende Knochengewebe unter der Wirkung des Formkegels 22 zusätzlich erweitert und durch Preßformung verdichtet. Während des Einschraubens dient die Nut 21 als Markierung für die erreichten Einschraubtiefe. Neben oder anstatt der Nut 21 können mehrere Markierungsnuten vorgesehen werden, die im Bereich der Formkegel 20 und 22 angebracht werden.

Das so vorbehandelte Knochengewebe ermöglicht eine enge Verbindung zwischen Knochen und Implantat, das nach Entfernen der Formschraube in das so vorbehandelte Loch im Knochen eingebracht wird. Hierdurch werden von Anfang an ein sicherer Sitz des Implantats erreicht und ein problemloses Einheilen begünstigt.

Die Formschraube kann für Zahnimplantate verwendet werden, ist aber nicht auf diese beschränkt, sondern ist für Knochenimplantate unterschiedlichster Art geeignet, so z.B. für Implantate im Hüft-, Knie- und Schultergelenk, für Wirbelsäulenversteifungen, usw.

Bei der dargestellten Formschraube sind zwei selbstschneidende Gewindeabschnitte 7 und 17 vorgesehen. Die Erfindung ist nicht auf diese zahl begrenzt. Wenn das Implantat einen abgestuften Durchmesser mit mehr als zwei Stufen aufweist, kann die Formschraube eine gleiche Anzahl selbstschneidender Gewindeabschnitte aufweisen, die entsprechend dem Implantat in ihren Durchmessern gegeneinander abgestuft sind.

Während die Erfindung anhand bevorzugter Ausführungsformen beschrieben wurde, können Abwandlungen und andere Ausführungsformen realisiert werden, ohne dass dadurch der in den Ansprüchen definierte Bereich der Erfindung verlassen wird.

## Patentansprüche

1. Formschraube für Knochenimplantate, die vor dem Einsetzen eines Implantats in den menschlichen Knochen eingeschraubt wird zur Herstellung eines passgenauen Lagers für das in den Knochen einzusetzende Implantat und die einen in Abschnitte unterteilten Schaft (5) aufweist, der kopfseitig zum Eingriff mit einem Drehwerkzeug eingerichtet ist (26), **gekennzeichnet durch**
einen ersten zylindrischen Abschnitt (7), der an den Fuß (6) der Formschraube anschließt und ein selbstschneidendes Gewinde (8) aufweist;
wenigstens einen zweiten zylindrischen Abschnitt (17), der an den ersten Abschnitt anschließt und dessen Durchmesser größer ist als der des ersten Abschnitts und der ein Gewinde (18) aufweist, das wenigstens über einen Teil des zweiten Abschnitts selbstschneidend ausgebildet ist; und
wenigstens einen an den zweiten Abschnitt anschließenden kegelförmigen Abschnitt (20, 22), über dessen Länge sich der Durchmesser der Formschraube zum Schraubenkopf hin vergrößert und der beim Einschrauben das den kegelförmigen Abschnitt (20, 22) umgebende Knochengewebe verdichtet und erweitert.

2. Formschraube nach Anspruch 1, **dadurch gekennzeichnet, dass** zwischen dem ersten Abschnitt (7) und dem zweiten Abschnitt (17) ein abgeschrägter Übergangsbereich (9) angeordnet ist und dass sich das Gewinde (8, 18) auch über diesen Übergangsbereich erstreckt.

3. Formschraube Anspruch 1 oder 2, **gekennzeichnet durch** zwei aufeinanderfolgende Formkegel (20, 22) unterschiedlicher Steigung.

4. Formschraube nach Anspruch 3, **gekennzeichnet durch** einen ersten Formkegel (20), der sich an den Gewindeabschnitt (17) mit dem größten Durchmesser anschließt, und einen zweiten Formkegel (22), der dem ersten Formkegel (20) benachbart ist und einen größeren Neigungswinkel als dieser aufweist.

5. Formschraube nach Anspruche 3 oder 4, **dadurch gekennzeichnet, dass** im Bereich der Formkegel (20, 22) wenigstens eine Tiefenmarkierung (21) angebracht ist.

6. Formschraube nach einem der Ansprüche 3 bis 5, **dadurch gekennzeichnet, dass** an der Grundfläche des kopfseitigen Formkegels (22) einen Zapfen (25) angeordnet ist, der eine Einrichtung (26) zum Eingriff mit einem Drehwerkzeug für den Antrieb der Schraube aufweist.

## Claims

1. Forming screw for bone implants, which is screwed in before an implant is inserted into the human bone for generating a fitting precise bearing of the implant to be inserted into the bone, and which comprises a shaft (5) being divided into sections and adapted at its head-side to engage with a rotary tool (26), **characterized by**
a first cylindrical section (7), which is adjacent to the foot (6) of the forming screw and which comprises a self-cutting thread;
at least one second cylindrical section (17), located adjacent to the first cylindrical section (7) and having a diameter which is greater than the diameter of the first section and comprises a thread (18) that is formed as self-cutting thread at least over a portion of the second section is; and
at least one conical section (20, 22) located adjacent to the second section, by which the diameter of the forming screw increases over the length of the conical section in the direction of the screw head, and which compresses and extends the bone tissue surrounding the conical section (20, 22) when the forming screw is screwed in.

2. Forming screw according to claim 1, **characterized in that** a slanted transition area (9) is arranged between the first section (7) and the second section (17) and that the thread (8, 18) also extends over this transition area.

3. Forming screw according to claim 1 or 2, **characterized by** two successive forming cones (20, 22) having different inclination.

4. Forming screw according to claim 3, **characterized by** a first forming cone (20), located adjacent to the thread section (17) of the greatest diameter, and a second forming cone (22) which is adjacent to the first forming cone (20) and comprises a greater inclination angle than the latter.

5. Forming screw according to claim 3 or 4, **characterized in that** in the area of the forming cones (20, 22) there is arranged at least one depth marking (21).

6. Forming screw according to one of the claims 3 to 5, **characterized in that** a post (25) is arranged at the basic plane of the forming cone (22) located at the head-side of the screw; the post comprises a device (26) for engaging with a rotary tool for driving the screw.

## Revendications

1. Vis taraudeuse pour implants osseux qui est vissée avant la mise en place de l'implant dans l'os humain afin de réaliser un logement de précision pour l'implant à mettre en place dans l'os et qui présente une tige (5) subdivisée en tronçons qui est aménagée du côté de la tête pour permettre une intervention au moyen d'un outil de mise en rotation **caractérisée par**
un premier tronçon (7) cylindrique qui suit le pied (6) de la vis taraudeuse et qui présente un filet (8) autotaraudeur ;
au moins un second tronçon (17) cylindre qui suit le premier tronçon et dont le diamètre est plus grand que celui du premier tronçon et qui présente un filet (18) qui est conformé au moins sur une partie du second tronçon en filet autotaraudeur ; et
au moins un tronçon (20, 22) de forme conique qui suit le second tronçon, le long duquel le diamètre de la vis taraudeuse s'agrandit en allant vers la tête de vis et qui, lors du vissage, rend étanche et élargit le tissu osseux entourant le tronçon (20, 22) de forme conique.

2. Vis taraudeuse selon la revendication 1, **caractérisée en ce qu'**une zone de transition (9) biaisée est disposée entre le premier tronçon (7) et le second tronçon (17) et **en ce que** le filet (8, 18) s'étend aussi sur cette zone de transition.

3. Vis taraudeuse selon la revendication 1 ou 2 **caractérisée par** deux cônes taraudeurs (20, 22) de conicité différente qui se suivent.

4. Vis taraudeuse selon la revendication 3 **caractérisée par** un premier cône taraudeur (20) qui suit le tronçon de filet (17) au diamètre le plus grand, et un second cône taraudeur (22), qui est voisin du premier cône taraudeur (20) et qui présente un angle d'inclinaison plus grand que ce dernier.

5. Vis taraudeuse selon la revendication 3 ou 4 **caractérisée en ce qu'**au moins un marquage de profondeur (21) est mis en place au niveau des cônes taraudeurs (20, 22).

6. Vis taraudeuse selon une des revendications 3 à 5 **caractérisée en ce qu'**un tourillon (25) est placé sur la surface de base du cône taraudeur (22) du côté de la tête, qui présente un dispositif (26) pour une intervention avec un outil de mise en rotation pour entraîner la vis.
